# EUROPÄISCHE PATENTANMELDUNG

(11) **EP 0 664 106 A2**
(43) Veröffentlichungstag der Anmeldung: **26.07.1995**
(21) Anmeldenummer: 94115391.8
(22) Anmeldetag: 29.09.1994
(51) Int. Cl.: A61C 13/30

(54) **System zur Restauration zerstörter Zähne**

(30) Priorität: 11.10.1993 DE 4334608
(71) Anmelder: Gebr. Brasseler GmbH & Co. KG, D-32657 Lemgo (DE)
(72) Erfinder: Musil, Rudolf, Dr., D-07743 Jena (DE)
(74) Vertreter: Weber, Joachim, Dr.

(57) **Zusammenfassung**

Die Erfindung bezieht sich auf ein System zur Restauration zerstörter Zähne, mit
- zumindest einem Wurzelstift (1), welcher einen Schaft (2) aufweist, der in einem Kanal (10) einer Zahnwurzel (3) fixierbar ist,
- einem am oberen Endbereich des Wurzelstiftes (1) einstückig mit diesem ausgebildeten Retentionskopf (4),
- einer am Retentionskopf (4) verankerbaren Kappe (5), und
- einer auf die Kappe (5) aufbringbaren Verblendkrone (6),
wobei erfindungsgemäß vorgesehen ist,
- daß die Kappe (5) als vorkonfektioniertes Teil ausgebildet ist,
- daß die Kappe (5) mit einer bodenseitigen Montageausnehmung (7) versehen ist, in welche der Retentionskopf (4) mit Spiel einbringbar ist, und
- daß die Kappe (5) und der Retentionskopf (4) mittels einer Fixiermasse verbunden sind.

## Beschreibung

Die Erfindung bezieht sich auf ein System zur Restauration zerstörter Zähne.

Auf dem Gebiet der restaurativen Zahnheilkunde sind unterschiedlichste Vorgehensweisen bekannt, um koronal zerstörte, devitale oder wurzelbehandelte Zähne zu restaurieren. Bei den bekannten Verfahren wird nach einer mit Erfolg abgeschlossenen Wurzelbehandlung ein Wurzelstift in den Wurzelkanal des Zahns eingesetzt. Der Wurzelkanal muß dabei, abhängig von der Ausgestaltung des Wurzelstiftes entsprechend erweitert werden, damit der Wurzelstift fest sitzend fixiert werden kann. Die Fixierung erfolgt beispielsweise durch zahnärztliche Befestigungsmittel. Die Ausgestaltung der Schäfte der einzelnen Wurzelstifte ist unterschiedlich, es sind Wurzelstifte mit zylindrischen, konischen oder mit einem Gewinde versehenen Schäften aus dem Stand der Technik bekannt. Weiterhin ist es bekannt, ggf. zwei Wurzelstifte in die beiden Wurzeln eines mehrwurzligen Zahnes einzubringen.

An dem Wurzelstift wird, wie aus dem Stand der Technik ebenfalls bekannt ist, ein Aufbau erstellt, auf welchem wiederum eine Verblendkrone aufgebracht wird. Bei der Herstellung der Aufbauten ergeben sich die unterschiedlichsten Möglichkeiten:

Der koronale Aufbau kann plastisch modelliert werden, beispielsweise aus einem Kunststoff oder aus Amalgam. Amalgam ist jedoch wegen seiner möglicherweise gesundheitsheitsschädlichen Wirkungen kontraindiziert, bei der Verwendung von Kunststoffen können bei der Schrumpfung Spaltbildungen auftreten, welche u.a. zu Karies führen können. Ein weiterer Nachteil besteht darin, daß der Aufbau aus einem der genannten plastischen Materialien arbeitsaufwendig ist und in manchen Mundbereichen nur mit Schwierigkeiten durchgeführt werden kann.

Eine Variante zu der oben beschriebenen Vorgehensweise besteht darin, den Wurzelstift und den Aufbau einstückig auszubilden. Um jedoch hierbei die anatomischen Gegebenheiten berücksichtigen zu können, sind eine Vielzahl von Stiftkopfabmessungen bzw. Aufbauabmessungen erforderlich, weiterhin ist das Beschleifen der metallischen Aufbauten oder Stiftköpfe sehr arbeits- und zeitaufwendig.

Der Stand der Technik zeigt weiterhin Systeme, bei welchen ein Wurzelstift an der Zahnwurzel fixiert wird, auf welchen nachfolgend eine konfektionierte Aufbauhülse aufgebracht wird. Die Hülse wird dabei formschlüssig passend auf den Wurzelstift aufgeschoben oder aufgeschraubt und durch einen Klebstoff fixiert. Hierbei ist es jedoch erforderlich, eine Vielzahl unterschiedlichster Aufbauhülsen bereit zu halten, um die anatomischen Gegebenheiten zu berücksichtigen. Daraus ergibt sich der Nachteil, daß dieses System insgesamt sehr kostenaufwendig ist, da der Zahnarzt eine große Anzahl von Aufbauhülsen bereithalten muß. Dies ist auch dadurch bedingt, daß die Kappe exakt fluchtend auf den Wurzelstift aufgesetzt wird. Die anatomisch bedingte Ausrichtung des Wurzelstiftes in dem Wurzelkanal gibt hierbei auch die Stellung der Kappe vor. Eine Anpassung an die Form des Zahnes ist somit nur durch die Form der Kappe selbst möglich. Da die Kappen aus metallischen Werkstoffen gefertigt sind, ist ein hoher Bearbeitungsaufwand nicht zu vermeiden.

Weiterhin sind aus dem Stand der Technik gegossene Aufbauten bekannt, bei welchen der Aufbau an einen Stift angegossen oder einstückig mit dem Stift gegossen werden. Diese Techniken sind zwar sehr präzise aber auch sehr aufwendig und kostenintensiv, da die Herstellung des Gußobjektes nur im Dentallabor durchführbar ist, der zu restaurierende Zahn für die Dauer der Laborarbeit provisorisch versorgt werden muß und hierdurch eine zusätzliche Sitzung zur Eingliederung des Stiftes mit angegossenem Aufbau oder des einstückig gegossenen Stiftaufbaus notwendig ist.

Die US-PS 4,645,457 beschreibt einen Stand der Technik gemäß dem Oberbegriff des Hauptanspruchs. Hierbei erweist es sich als nachteilig, daß der Wurzelstift, welcher mit einem Gewinde und einer Verankerugsmutter versehen ist, in aufwendiger Weise angepaßt und eingesetzt werden muß. Erst nach diesem Arbeitsvorgang ist es möglich, mittels eines Verankerungsmaterials, welches die Mutter umschließt, die Kappe aufzubringen, welche eine Verblendkrone tragen kann.

Die GB-A-2 199 751 beschreibt vorgefertigte Kronen mit einem Komposit-Aufbau. Diese Kronen können in üblicher Weise durch einen Dentisten eingesetzt werden.

Es ist somit festzustellen, daß die bekannten Verfahren eine Vielzahl von Nachteilen aufweisen. Die Hauptnachteile liegen darin, daß es mit einem erheblichen Aufwand verbunden ist, einen anatomisch "passenden" Aufbau auszubilden. Entweder ist hierzu eine große Anzahl vorgefertigter Aufbauhülsen erforderlich, oder es muß ein erheblicher manueller Aufwand betrieben werden. Zusätzlich sind einige Aufbauhülsen der beschriebenen Arbeiten nicht in einer Sitzung zu realisieren, so daß der Zahnarzt zwei Sitzungen benötigt. Weiterhin ist für den Zeitraum zwischen den beiden Sitzungen eine provisorische Versorgung erforderlich.

Der Erfindung liegt die Aufgabe zugrunde, ein System der eingangs genannten Art zu schaffen, welches bei einfachem Aufbau und einfacher, sicherer Handhabbarkeit den Zeit- und Arbeitsaufwand von koronal zerstörten Zähnen minimiert und bei guter Verträglichkeit für den Patienten kostengünstig ausgestaltet ist.

Erfindungsgemäß wird die Aufgabe durch die Merkmale des Hauptanspruchs gelöst, die Unteransprüche zeigen weitere vorteilhafte Ausgestaltungen der Erfindung.

Das erfindungsgemäße System zeichnet sich durch eine Reihe erheblicher Vorteile aus:
Da erfindungsgemäß die Kappe als vorkonfektioniertes Teil ausgebildet ist, kann ein sehr breites Anwendungsfeld unter Verwendung nur weniger Kappenformen abgedeckt werden. Somit vereinfacht sich die Lagerhaltung beim Zahnarzt und es ist sichergestellt, daß stets eine den Anforderungen genügende Kappe zur Verfügung steht. Die erfindungsgemäße Ausgestaltung der Kappe gestattet es, diese den jeweiligen anatomischen Gegebenheiten anzupassen, d.h. entsprechend auszurichten und einzupassen. Da die bodenseitige Montageausnehmung, in welcher der Retentionskopf angeordnet ist, ein ausreichendes Spiel aufweist, kann die Kappe praktisch beliebig gedreht oder geschwenkt werden, bevor sie an dem Wurzelstift fixiert wird.

Besonders günstig ist es dabei, daß die Längsachse der Kappe zur Achse des Wurzelstiftes geneigt angeordnet werden kann. Somit können anatomische Ungleichmäßigkeiten ausgeglichen werden, ohne daß arbeits- und zeitaufwendige Nacharbeiten an der Kappe erforderlich wären.

Im Vergleich zu gegossenen Zahnaufbauten entfällt eine provisorische Versorgung, da der Aufbau in einer Sitzung herstellbar ist.

Erfindungsgemäß kann die Fixiermasse in Form eines härtbaren oder härtenden Kunststoffmaterials ausgebildet sein. Dieses Material kann beispielsweise lichtaushärtend sein, es ist jedoch auch möglich, einen auto-aushärtenden Kunststoff auf der Basis einer Zwei-Komponenten-Mischung zu verwenden.

Erfindungsgemäß ergibt sich somit der Vorteil, daß die Fixiermasse an den Schnittstellen Keramik-Füllmasse und Füllmassen-Beschichtung des Retensionskopfes des Wurzelstiftes einen chemisch spaltfreien Verbund eingeht.

Erfindungsgemäß kann die Kappe aus Kunststoff, aus einem metallischen Werkstoff oder einem keramischen Werkstoff gefertigt sein. Bei der Herstellung aus Keramik ergeben sich weitere, zusätzliche Vorteile. Standardisierte Kappenformen aus Keramik sind einfach und kostengünstig herstellbar, das Keramik-Material ist aus dem Stand der Technik bekannt und auf dem Medizinsektor vielfach erprobt. Die Keramik kann biokompatibel sein, es ist auch möglich, ein bioaktives Keramik bioaktive Stoffe wie zum Beispiel Hydroxylapatit-Kristalle eingelagert sind.

Erfindungsgemäß können die Keramik-Kappen auf unterschiedlichste Weise hergestellt werden. Beispielsweise werden die Bestandteile des Werkstoffes oberhalb 1450° C geschmolzen, gegossen und abgeschreckt. Danach wird nochmals erwärmt und abgeschreckt. Die dabei herstellbaren Blöcke können mechanisch zu kleineren Einheiten zertrennt werden, anschließend werden die Halbzeuge keramisiert. Bei der Herstellung handelt es sich somit um einen Bi-Hochtemperaturprozeß. Weitere Herstellungsmöglichkeiten für die Halbzeuge sind das Pressen des Schmelzgutes in Formen oder das Stranggießen oder das Sintern. Es ist somit offensichtlich, daß die Glaskeramik-Kappen kostengünstig und einfach in standardisierten Abmessungen, welche für eine große Anzahl von Standardsituationen verwendbar sind, hergestellt werden können.

Die Keramik-Materialien sind weiterhin hinsichtlich ihrer Werkstoffeigenschaften, wie Härte, Festigkeit, E-Modul, besonders vorteilhaft. Weiterhin können die Keramik-Materialien schnell und problemlos mittels Diamant-Schleifinstrumenten präpariert werden. Im Vergleich zu metallischen Werkstoffen ergibt sich hieraus eine ganz wesentliche Ersparnis hinsichtlich des Arbeitsaufwandes und der benötigten Bearbeitungszeit.

Weiterhin ist es günstig, daß Keramikwerkstoffe zahnfarben sein können, so daß die Zahnaufbauten auch höchsten optischen Anforderungen genügen. Dies ist insbesondere bei Verwendung von Jacketkronen besonders vorteilhaft.

Im Vergleich mit aus dem Stand der Technik bekannten Kunststoff- oder Amalgamaufbauten ist zu bemerken, daß ein Keramik-Stumpf des Zahnaufbaus einen sehr stabilen Körper bildet, welcher weit höher belastbar ist.

Als weiterer Vorteil ist zu erwähnen, daß die erfindungsgemäß vorgesehenen Keramikkappen, im Vergleich zu Kunststoffaufbauten weit weniger elastisch sind. Dadurch erhöht sich die Lebensdauer ganz erheblich, da unter Kaubelastung keine Spaltbildung zwischen der Wurzelstumpfoberfläche und dem Aufbaumaterial entsteht.

Das erfindungsgemäße System ist insbesondere für tiefzerstörte und querfrakturierte Zähne besonders günstig einsetzbar. Hierbei ist insbesondere darauf zu verweisen, daß bei dem erfindungsgemäßen System handelsübliche Wurzelstifte verwendet werden können.

Der Behandlungsablauf gestaltet sich bei Verwendung des erfindungsgemäßen Systems in folgender Weise:
- Endodontische Aufbereitung
- Präparation/Erweiterung des Wurzelkanals
- Applikation des Wurzelstiftes
- Aufbringen und Fixieren der Kappe
- Präparation der Kappe
- Aufbringung der Verblendkrone.

Im folgenden wird die Erfindung anhand von Ausführungsbeispielen in Verbindung mit der Zeichnung beschrieben. Dabei zeigt:
- Fig. 1: eine schematische Darstellung des fertig applizierten erfindungsgemäßen Systems an einer Zahnwurzel,
- Fig. 2: eine Seiten-Schnittansicht (Sagittalschnitt) eines ersten Ausführungsbeispiels einer erfindungsgemäßen Kappe,
- Fig. 3: eine Frontalansicht/Seitenansicht der in Fig. 2 gezeigten Kappe,
- Fig. 4: eine Draufsicht auf die in Fig. 2 und 3 gezeigte Kappe,
- Fig. 5: eine Schnittansicht (Frontalschnitt) eines weiteren Ausführungsbeispiels der erfindungsgemäßen Kappe zur Verwendung bei Molaren,
- Fig. 6: eine Seitenansicht (Sagittalschnitt) der in Fig. 5 gezeigten Kappe, und
- Fig. 7: eine Draufsicht auf die in Fig. 5 und 6 gezeigte Kappe.

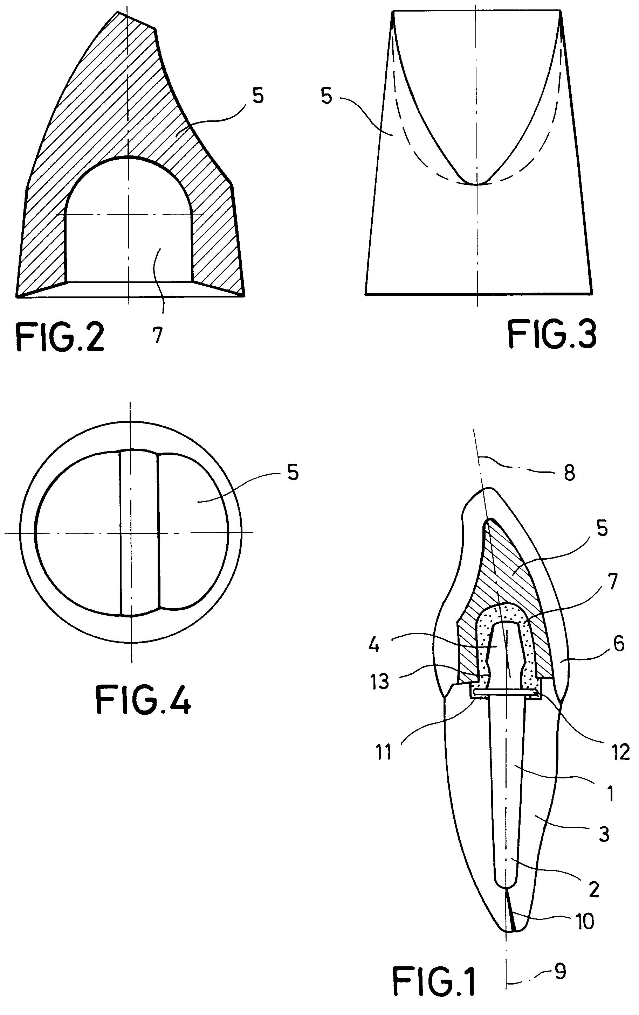

Die Fig. 1 zeigt in der Seitenansicht in schematischer Darstellung eine Zahnwurzel 3, welche einen schematisch dargestellten Wurzelkanal 10 aufweist. Der Wurzelkanal 10 wurde durch Bohren oder Fräsen so präpariert, daß ein Schaft 2 eines Wurzelstiftes 1 paßgenau bzw. formschlüssig eingesetzt werden konnte. Der Schaft 2 ist im gezeigten Ausführungsbeispiel konisch ausgebildet, seine Längsachse 9 erstreckt sich im wesentlichen längs des Wurzelkanals 10. Die Fixierung des Schaftes 2 erfolgt mittels eines üblichen Zements oder eines ähnlichen Werkstoffs.

Die Fig. 1 zeigt weiterhin, daß am oberen Bereich des Wurzelkanals 10 eine Anlagefläche 11 ausgefräst wurde, gegen welche eine scheibenförmige, einstückig mit dem Wurzelstift 1 ausgebildete Schulter 12 in Anlage kommt.

Der Wurzelstift 1 trägt an seinem oberen Ende einen Retentionskopf 4, welcher mit einem Hinterschnitt 13 versehen ist. Der Retentionskopf 4 kann mit einer haftvermittelnden Schicht (PCR-Schicht zum Beispiel) versehen sein.

Erfindungsgemäß ist weiterhin eine Kappe 5 aus einem Glaskeramik-Werkstoff vorgesehen, welche eine bodenseitige Montageausnehmung 7 aufweist. Die Montageausnehmung 7 ist so dimensioniert, daß der Retentionskopf 4 mit Spiel aufnehmbar ist. Hierdurch ist es möglich, die Kappe 5 in geeigneter Weise auszurichten. Die Längsachse 8 der Kappe 5 kann beispielsweise zu der Längsachse 9 des Wurzelstiftes 1 geneigt werden. Hierdurch ist eine besonders einfache Anpassung an anatomische Gegebenheiten möglich.

Die Fixierung der Kappe 5 an dem Retentionskopf 4 erfolgt mittels einer gepunktet gezeichneten Fixiermasse, welche in Form eines aushärtenden oder aushärtbaren Kunststoffmaterials ausgebildet ist. Da die Fixiermasse zähflüssig ist, kann die Aushärtung in einfacher Weise erfolgen, nach dem der Zahnarzt die Kappe 5 in ihre Lage eingestellt und ausgerichtet hat.

Auf die Kappe 5 wird eine Verblendkrone 6 aufgesetzt.

Da erfindungsgemäß eine ausreichende Anzahl vorkonfektionierter Kappen 5 vorgesehen ist und da die jeweilige Kappe 5 gegenüber dem Retentionskopf 4 in einfacher Weise ausgerichtet und eingestellt werden kann, ist nur ein sehr geringer Nacharbeitsaufwand für die Anpassung der Form der Kappe 5 erforderlich. Diese nachfolgende Bearbeitung erfolgt bevorzugterweise mittels Diamantwerkzeugen. Eine derartige Bearbeitung ist schnell und problemlos durchführbar.

Als Material für die Kappe 5 ist beispielsweise ein Glaskeramikwerkstoff verwendbar. Die technologischen Eigenschaften dieses Werkstoffes sind aus dem Stand der Technik bekannt, so daß auf eine detaillierte Beschreibung an dieser Stelle verzichtet werden kann.

Die Verblendkrone 6 wird nach Anpassung der Form der Kappe 5 aufgrund eines Abdrucks im Labor gefertigt und nachfolgend, wie ebenfalls aus dem Stand der Technik bekannt, aufgesetzt.

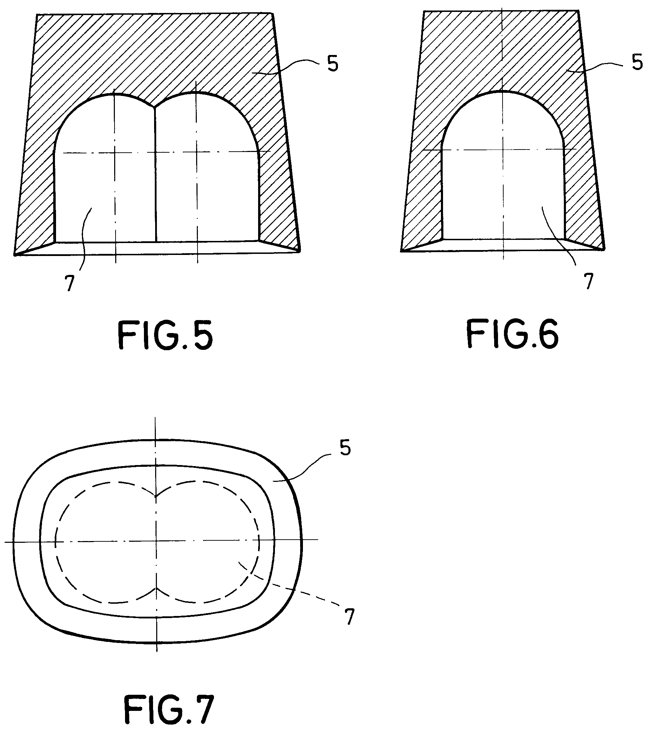

Die Fig. 2 bis 4 bzw. 5 bis 7 zeigen jeweils unterschiedliche Ausgestaltungen von Standardformen der erfindungsgemäßen Kappe 5. Wie bereits beschrieben, kann in einfachster Weise eine Anpassung an den jeweils zu restaurierenden Zahn erfolgen. Die Kappe 5 weist, wie bereits erwähnt, an ihrer Bodenseite die Montageausnehmung 7 auf. Diese ist, wie insbesondere aus den Fig. 2 und 6 zu ersehen ist, zylindrisch und endet in einer halbkugelförmigen Kalotte. Während die Kappe der Fig. 2 bis 4 so ausgebildet ist, daß sie auf einen Wurzelstift aufsetzbar ist, bietet die Kappe der Fig. 5 bis 7 die Möglichkeit, beide Zahnwurzeln zu nutzen, um zwei Wurzelstifte zu verankern. Somit können zwei Retentionsköpfe in der Montageausnehmung 7 aufgenommen werden. Hierbei ergibt sich der besondere Vorteil, daß die jeweilige Lage der Retentionsköpfe, welche durch die Wurzelkanäle bedingt ist, beim Aufsetzen der Kappe entsprechend ausgeglichen werden kann, so daß nur geringe Nacharbeiten an der Kappe selbst erforderlich sind.

## Patentansprüche

1. System zur Restauration zerstörter Zähne, mit
- zumindest einem Wurzelstift (1), welcher einen Schaft (2) aufweist, der in einem Kanal (10) einer Zahnwurzel (3) fixierbar ist,
- einem am oberen Endbereich des Wurzelstiftes (1) einstückig mit diesem ausgebildeten Retentionskopf (4),
- einer am Retentionskopf (4) verankerbaren Kappe (5), und
- einer auf die Kappe (5) aufbringbaren Verblendkrone (6),
dadurch gekennzeichnet,
- daß die Kappe (5) als vorkonfektioniertes Teil ausgebildet ist,
- daß die Kappe (5) mit einer bodenseitigen Montageausnehmung (7) versehen ist, in welche der Retentionskopf (4) mit Spiel einbringbar ist, und
- daß die Kappe (5) und der Retentionskopf (4) mittels einer Fixiermasse verbunden sind.

2. System nach Anspruch 1, dadurch gekennzeichnet, daß die Längsachse (8) der Kappe (5) zur Achse (9) des Wurzelstiftes (1) geneigt anzuordnen ist.

3. System nach Anspruch 1 oder 2, dadurch gekennzeichnet, daß die Kappe (5) in jeweils unterschiedlichen Außenabmessungen gefertigt ist.

4. System nach Anspruch 3, dadurch gekennzeichnet, daß die Fixiermasse in Form eines härtbaren oder härtenden Befestigungsmaterials ausgebildet ist.

5. System nach einem der Ansprüche 1 bis 4, dadurch gekennzeichnet, daß die Kappe aus Kunststoff gefertigt ist.

6. System nach einem der Ansprüche 1 bis 4, dadurch gekennzeichnet, daß Kappe (5) aus einem metallischen Werkstoff gefertigt ist.

7. System nach einem der Ansprüche 1 bis 4, dadurch gekennzeichnet, daß die Kappe (5) aus einem Keramikwerkstoff gefertigt ist.

8. System nach Anspruch 7, dadurch gekennzeichnet, daß der Keramikwerkstoff biokompatibel ist.

9. System nach Anspruch 7, dadurch gekennzeichnet, daß der Keramikwerkstoff in die Matrix eingelagerte Hydroxylapatit-Kristalle aufweist und bioaktiv ist.

10. System nach einem der Ansprüche 1 bis 9, dadurch gekennzeichnet, daß mehrere Retentionsköpfe (4) mehrerer Wurzelstifte (1) in die Montageausnehmung (7) einbringbar sind.
